(19) Europäisches Patentamt
European Patent Office
Office européen des brevets

(11) Veröffentlichungsnummer: **0 065 629**
**B1**

(12) **EUROPÄISCHE PATENTSCHRIFT**

(45) Veröffentlichungstag der Patentschrift: **29.05.85**

(51) Int. Cl.⁴: **C 07 C 69/24**, C 07 C 67/38

(21) Anmeldenummer: **82102144.1**

(22) Anmeldetag: **17.03.82**

(54) **Verfahren zur kontinuierlichen Herstellung von Alkylestern gesättigter aliphatischer Carbonsäuren.**

(30) Priorität: **16.05.81 DE 3119594**

(43) Veröffentlichungstag der Anmeldung: **01.12.82 Patentblatt 82/48**

(45) Bekanntmachung des Hinweises auf die Patenterteilung: **29.05.85 Patentblatt 85/22**

(84) Benannte Vertragsstaaten: **AT BE FR GB IT NL**

(56) Entgegenhaltungen: **EP - A - 0 017 051**

(73) Patentinhaber: **CHEMISCHE WERKE HÜLS AG, - RSP Patente / PB 15 - Postfach 13 20, D-4370 Marl 1 (DE)**

(72) Erfinder: **Hofmann, Peter, Dr., Lipper Weg 193, D-4370 Marl (DE)**
Erfinder: **Regner, Hans, Dr., Griesheimer Strasse 7, D-4370 Marl (DE)**
Erfinder: **Köppner, Manfred, Dr., Bitterfelder Strasse 7 a, D-4370 Marl (DE)**
Erfinder: **Zölffel, Michael, Hoechster Strasse 20, D-4370 Marl (DE)**

**EP 0 065 629 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es ist bekannt, daß man durch Umsetzung von Olefinen mit Kohlenmonoxid und H-aciden Komponenten, wie z. B. Alkanolen, in Gegenwart eines Katalysators, der ein Metall der 8. Nebengruppe des Periodischen Systems der Elemente und gegebenenfalls einen Promotor enthält, Fettsäureester herstellen kann (J. Falbe, Synthesen mit Kohlenmonoxid, Springer-Verlag, Berlin, Heidelberg, New York, 1967).

Eine bevorzugte Variante dieser als Hydrocarboxilierung bezeichneten Reaktion ist die Umsetzung in Gegenwart von cobalthaltigen Katalysatoren. Als besonders bevorzugte Ausführungsform hat sich der zusätzliche Einsatz von Pyridin oder einem nicht-ortho-substituierten Alkylpyridin als Promotor erwiesen (US-PS 3 507 891 und DE-OS 2 912 489).

Die durch Cobalt/Pyridin oder Pyridinderivate katalysierte Hydrocarboxilierungsreaktion ähnelt hinsichtlich ihres Chemismus, der für die Umsetzung erforderlichen Einsatzstoffe, der Reaktionsbedingungen sowie der freiwerdenden Reaktionswärme anderen Carbonylierungsreaktionen, insbesondere der Hydroformylierung von Olefinen.

So unterscheiden sich die Katalysezyklen von Hydrocarboxilierung und Hydroformylierung nur im letzten Schritt bei der Bildung des Endproduktes. Während die Aldehydbildung durch Hydrogenolyse der C—Co-Bindung des Acylkomplexes erfolgt, findet die Esterbildung durch Alkoholyse dieser Bindung statt:

$$R—CH{=}CH_2 + HCo(CO)_xL_y \longrightarrow R—CH_2CH_2—Co(CO)_xL_y \longrightarrow$$

$$R—CH_2—CH_2—CHO + HCo(CO)_{x-1}L_y$$

$$\uparrow + H_2$$

$$\longrightarrow R—CH_2—CH_2—CO—Co(CO)_{x-1}L_y$$

$$\downarrow + CH_3OH$$

$$R—CH_2—CH_2—COOCH_3 + HCo(CO)_{x-1}L_y$$

$$HCo(CO)_{x-1}L_y + CO \longrightarrow HCo(CO)_xL_y$$

($x+y=3$ oder 4; L = CO oder Pyridin bzw. Pyridinderivat)

Die Einsatzstoffgemische von Hydrocarboxilierung und Hydroformylierung bestehen also in der Regel aus Komponenten, die unter dem für die Reaktion angewandten Druck flüssig vorliegen und solchen, die auch unter diesen Bedingungen noch gasförmig sind.

Hinsichtlich optimaler Druck- und Temperaturbereiche bestehen zwischen der durch Cobalt und Pyridin bzw. Pyridinderivaten katalysierten Hydrocarboxilierung und der durch das gleiche Metall katalysierten Hydroformylierung kaum Unterschiede. In beiden Fällen liegen die üblicherweise eingehaltenen Temperaturen und Drucke im Bereich von 130 bis 230° C bzw. 100 bis 300 bar.

Beide Reaktionen sind exotherm; die jeweiligen Reaktionswärmen liegen im allgemeinen in einem Bereich von 28 bis 35 kcal/Mol.

Entsprechend dieser weitgehenden Analogie beider Reaktionen ist es nicht verwunderlich, daß für die kontinuierlich durchgeführte, großtechnische Realisierung von Hydrocarboxilierung und Hydroformylierung identische Reaktorkonzepte vorgeschlagen werden (DE-PS 926 846, Seite 2, Zeilen 95 bis 117). Es handelt sich dabei in der Regel um zylindrische Reaktionsgefäße, in denen durch besondere Einbauten — wie z. B. konzentrische Leitrohre — und die spezielle Art der Einbringung der Einsatzstoffe — z. B. Eindüsung mit hoher Geschwindigkeit an ein oder mehreren Stellen des Reaktors — eine intensive Durchmischung des Reaktorinhalts erreicht wird. Es ist eine ganze Reihe von Maßnahmen bekannt, mit Hilfe derer die für Carbonylierungsreaktoren typische Rührkesselcharakteristik [H. Dubil, J. Gaube, Chem. Ing. Techn. 45 (8), 529—533 (1973)] erreicht wird: Nach dem Verfahren der DE-AS 1 135 879 wird ein Flüssigkeitsumlauf durch Wärmekonvektion um ein im Reaktorinneren frei stehendes Umlaufrohr eingestellt. Bei dem Verfahren der DE-AS 1 085 144 erreicht man den Kreislauf des Reaktorinhalts durch hohe Gasbelastung nach dem Prinzip der Mammutpumpe. Gemäß dem Verfahren der DE-AS 1 205 514 wird Flüssigkeitsumlauf durch Impulseintrag der mit hoher Geschwindigkeit eingedüsten flüssigen Reaktionsteilnehmer bewirkt. Ebenfalls Rührkesselverhalten läßt sich nach dem Verfahren der DE-PS 1 003 708 durch Umpumpen des flüssigen Reaktorinhalts einstellen.

Obwohl als Folge des für Rührkessel charakteristischen Verweilzeitverhaltens bei Einhaltung des obigen Reaktorkonzeptes ohne besondere Zusatzmaßnahmen stets Umsatzeinbußen in Kauf zu nehmen sind, hat sich der Reaktor mit Rückvermischung als eine gebräuchliche Lösung für die Durchführung von Carbonylierungsreaktionen in der Technik durchgesetzt (vgl. auch Herstellung von Essigsäure durch Carbonylierung von Methanol, J. Falbe, Synthesen mit Kohlenmonoxid, Springer Verlag, Berlin, Heidelberg, New York, 1967, Seite 118). Die in diesem Reaktor gegebenen Wärmeübertragungsverhältnisse (höhere Strömungsgeschwindigkeit an den Kühlflächen und dadurch bessere Beherrschung der abzuführenden Reaktionswärme) gestatten im Zusammenwirken mit der Rückvermischung größtmögliche Temperaturkonstanz im gesamten Reaktionsraum. Bezüglich der Reaktionstemperatur werden definierte Reaktionsbedingungen als Voraussetzung für optimale Selektivität der Carbonylierungsreaktion angesehen; und nur so erscheint im Falle der Bildung mehrerer isomerer Produkte das optimale Isomerenverhältnis einstellbar. Eine gleichmäßige Temperatur soll schließlich eine in Zonen lokaler Überhitzung mögliche Katalysatordesaktivierung und zugleich ein »Einschlafen« der Reaktion an Stellen zu niedriger Temperatur verhindern. Umsatzeinbußen sowie in konstruktiver Hinsicht aufwendige Reaktoren müssen für die obengenannten Vorteile in Kauf genommen werden.

In der US-PS 3 976 670 wird schließlich ein Reaktorkonzept zur kontinuierlichen Durchführung der Hydrocarboxilierungsreaktion vorgeschlagen, das es gestattet, auch ohne Rückvermischung das Problem der Wärmeabfuhr zu lösen. Die Reaktion wird nach diesem Verfahren in einem Reaktor mit mehreren Zwischeneinspeisungen durchgeführt, der aus mehreren Segmenten mit gegen Ende des Reaktionsrohres zunehmendem Durchmesser besteht. Das Problem der Wärmeabfuhr wird dadurch gelöst, daß das Alkanol nicht nur am Beginn des Reaktors zusammen mit den anderen Reaktionsteilnehmern eingesetzt, sondern längs des Reaktors noch zusätzlich an mehreren Stellen gleichzeitig zudosiert wird. Durch das stets begrenzte lokale Alkanolangebot soll ein gleichmäßigerer Reaktionsablauf längs des gesamten Reaktors und damit eine gleichmäßigere Wärmeabfuhr sowie eine längere Lebensdauer des Katalysators erreicht werden.

Dem stehen, bedingt durch die besondere Art der Reaktionsführung, folgende Nachteile gegenüber: (1) Hohes Investment für einen aus mehreren Segmenten unterschiedlichen Durchmessers bestehenden Hochdruckreaktor. (2) Hoher Regelaufwand für die Dosierung mehrerer Stoffströme. (3) Im Bereich kürzerer, für eine technische Realisierung aus Kapazitätsgründen besonders interessanten Verweilzeiten, liegen die nach dem Verfahren der US-PS 3 976 670 erzielbaren Umsätze deutlich unter den durch diskontinuierliche Ansätze in Rührautoklaven bei gleichzeitiger Vorlage aller Reaktanten möglichen Werten (siehe US-PS 3 976 670, Beispiele 2 und 3, Versuche mit Zugabe der einfachen bzw. doppelten stöchiometrischen Alkoholmenge). Die sich möglicherweise bei dem Verfahren der US-PS 3 976 670 für längere Verweilzeiten ergebenden vergleichsweise höheren Umsätze sind wirtschaftlich nicht interessant, da bei einer solchen Verfahrensweise die Kapazität deutlich eingeschränkt würde (vgl. hierzu DE-OS 1 963 804, Seite 14, Abschnitt 2). (4) Schließlich ist die Anwendung des Verfahrens gemäß der US-PS 3 976 670 unweigerlich mit einer deutlichen Selektivitätseinbuße verbunden. Eine Begrenzung des lokalen Alkanolangebots führt nämlich infolge der zur Erzielung ausreichender Reaktionsgeschwindigkeiten erforderlichen Anwesenheit geringer Wasserstoffmengen (vgl. z. B. DE-OS 1 963 804, Anspruch 1; US-PS 3 507 891, Spalte 3, Zeilen 63 bis 67) zu einer Zunahme der durch Hydroformylierung als Nebenprodukte gebildeten Aldehyde und Acetale (vgl. hierzu P. Hofmann, K. Kosswig, W. Schäfer, Ind. Eng. Chem. Prod. Res. Dev., 1980, 19 330—334).

Reaktoren, die eine kontinuierlichen, großtechnische Realisierung exothermer Carbonylierungsprozesse gestatten, sind also, wie vorstehend angezeigt, weitgehend unter dem Gesichtspunkt optimaler Wärmeabfuhr konzipiert. Hierfür müssen notgedrungen die oben geschilderten Nachteile in Kauf genommen werden.

Aufgabe der vorliegenden Erfindung war es daher, ein Verfahren zu entwickeln, das es gestattet, Alkylester gesättigter aliphatischer Carbonsäuren durch Umsetzung von Olefinen mit mindestens 8 C-Atomen mit Kohlenmonoxid und Alkanol in Gegenwart eines Katalysators, bestehend aus einer Cobaltverbindung und einem Promotor aus der Gruppe Pyridin und/oder nicht-ortho-substituierter Alkylpyridine, bei erhöhtem Druck und erhöhter Temperatur so herzustellen, daß ein optimaler Kompromiß bezüglich der den wirtschaftlichen Erfolg des Hydrocarboxilierungsprozesses bestimmenden Zielgrößen — Umsatz, Selektivität und Isomerenverteilung — erreicht wird.

Diese Aufgabe wurde überraschenderweise durch die in den Ansprüchen beschriebenen Maßnahmen gelöst.

Überraschend deshalb, weil unter den erfindungsgemäßen Bedingungen keine, über das übliche Maß hinausgehenden Anstrengungen zur Wärmeabfuhr und Einhaltung einer konstanten Reaktionstemperatur unternommen werden müssen, wie dies bei den Verfahren des Standes der Technik zum Teil unter erheblichem technischen Aufwand und unter Inkaufnahme schwerwiegender Nachteile geschieht. Der Erfolg des erfindungsgemäßen Verfahrens, der unter anderem auf der Durchführung der Reaktion in einem Reaktor beruhrt, in dem sich die Strömungscharakteristik so einstellen läßt, daß Rohrströmung ohne Rückvermischung vorherrscht, ist weiterhin deshalb überraschend, weil er im Widerspruch zu der in der überwiegenden Zahl von Veröffentlichungen vertretenen Lehrmeinung — Durchführung der Carbonylierungsreaktion unter optimaler Rückvermischung — zustande kommt.

Das erfindungsgemäße Verfahren kann im Prinzip bei allen Hydrocarboxilierungsverfahren zur Herstellung von Carbonsäurealkylestern angewandt werden, bei denen ein Katalysator, bestehend aus einer Cobaltverbindung und Pyridin und/oder einem nicht-ortho-substituierten Alkylpyridin eingesetzt wird (z. B. Verfahren gemäß der US-PS 3 507 891 und der DE-OS 2 912 489). So ist vor allem die Wahl

des eingesetzten Olefintyps unkritisch, d. h. es können sowohl geradkettige oder verzweigte α-Olefine als auch Olefine mit innenständiger Doppelbindung eingesetzt werden, sofern sie mindestens 8 C-Atome besitzen.

Es können Olefine mit 8 bis 40, vorzugsweise mit 8 bis 20, besonders bevorzugt mit 10 bis 18 Kohlenstoffatomen, eingesetzt werden, die nach bekannten Verfahren des Standes der Technik erhalten werden können. So können z. B. α-Olefine durch Oligomerisierung von Ethylen nach Ziegler (DE-PS 878 560 und 1 190 930) oder durch Wachscracken, Olefine mit innenständiger Doppelbindung durch Dehydrierung oder Chlorierung und anschließender Dehydrochlorierung von Paraffinen (GB-PS 1 037 868) gewonnen werden.

Bei den von Paraffinen ausgehenden Verfahren werden in der Regel Paraffinschnitte eingesetzt, so daß auch die erhaltenen Olefine keine einheitliche C-Zahl aufweisen. Außerdem kommen in diesen Olefin-Gemischen natürlich alle denkbaren isomeren Formen vor.

Neben den reinen — gegebenenfalls substituierten — Olefinen können auch solche mit einem Gehalt an Paraffinen, z. B. bis zu 85 Gewichtsprozent, eingesetzt werden. Der Paraffingehalt rührt daher, daß bei der Olefinherstellung keine vollständigen Umsätze erreicht und die nicht umgesetzten Paraffine nicht oder nicht vollständig abgetrennt werden.

Nicht nur der Typ des Olefins, sondern auch der des Alkanols ist für das erfindungsgemäße Verfahren unkritisch. So können sowohl primäre, sekundäre als auch tertiäre Alkanole mit 1 bis 20, vorzugsweise primäre Alkanole mit 1 bis 3 Kohlenstoffatomen, wie z. B. Methanol, Ethanol und Propanol-(1), eingesetzt werden.

Für das erfindungsgemäße Verfahren ist es nicht kritisch, welche Cobaltverbindung zusammen mit dem Promotor als Katalysator eingesetzt wird. Carbonyle des Cobalts, z. B. Dicobaltoctacarbonyl, sind ebenso geeignet wie carbonsaure Cobaltsalze, wie z. B. Cobaltacetat, Cobaltnaphthenat und Cobalt-2-ethylhexanoat, und Salze des Cobalts mit organischen Säuren, wie z. B. Cobaltnitrat und Cobaltsulfat. Vorzugsweise kommen solche carbonsauren Cobaltsalze zum Einsatz, deren Anionen dem Säurerest der bei der Hydrocarboxilierung gebildeten Carbonsäurealkylester entsprechen.

Als Promotoren kommen neben Pyridin alle nicht-ortho-substituierten Alkylpyridine, wie z. B. 3- und 4-Picolin, 3,4- und 3,5-Dimethylpyridin und 3- und 4-Ethylpyridin allein oder im Gemisch infrage.

Es ist zweckmäßig, die Reaktionsbedingungen der Hydrocarboxilierung je nach Typ des zum Einsatz gelangenden Olefins zu variieren (P. Hofmann, K. Kosswig, W. Schäfer, Ind. Eng. Chem. Prod. Res. Dev., 1980, 19, 330—334). Unabhängig von der Art des Einsatzolefins liegt die Reaktionstemperatur im allgemeinen im Bereich von 80 bis 300, vorzugsweise 130 bis 230°C, der Kohlenmonoxiddruck im Bereich vom 10 bis 800, vorzugsweise 100 bis 300 bar.

Es ist ferner zweckmäßig, für die Einsatzstoffe mit Ausnahme von Kohlenmonoxid folgende Einsatzstoffverhältnisse einzuhalten:

Cobalt:
Olefin von 0,002 bis 0,3, vorzugsweise 0,005 bis 0,1 Grammatom Co/Mol Olefin;
Promotor:
Cobalt von 2 bis 100, vorzugsweise von 5 bis 50 Mole Promotor/Grammatom Co;
Alkanol:
Olefin von 1 bis 20, vorzugsweise von 1 bis 10 Mole Alkanol/Mol Olefin.

Kohlenmonoxid, das bis zu 10, vorzugsweise bis zu 5 Volumenprozent Wasserstoff enthalten kann, wird zweckmäßigerweise in einer solchen Menge zusammen mit den bereits genannten Einsatzstoffen zugeführt, daß die durch Reaktion und gegebenenfalls durch Abgasentnahme entstehenden Gasverbräuche kompensiert werden. Im allgemeinen betreibt man das erfindungsgemäße Verfahren wenigstens mit der stöchiometrischen, höchstens mit der doppelten stöchiometrischen Menge an Kohlenmonoxid, bezogen auf umgesetztes Olefin.

Die Einsatzstoffe werden von Anfang an gemäß dem Verfahren EP-A 0 017 051 im gewünschten bzw. erforderlichen Verhältnis eingespeist. Diese Maßnahme schließt die Durchführung der Hydrocarboxilierung in einem solchen Reaktor aus, bei dem ein oder mehrere Einsatzstoffe längs des Reaktors an Stellen unterschiedlichen Umsatzes eingespeist werden, wie z. B. gemäß dem Verfahren der US-PS 3 976 670.

Schließlich ist bei dem erfindungsgemäßen Verfahren die Teilmaßnahme kritisch, daß die kontinuierliche Hydrocarboxilierungsreaktion in einem Reaktor durchgeführt wird, in dem sich die Strömungscharakteristik so einstellen läßt, daß Rohrströmung ohne Rückvermischung vorherrscht. Das heißt, in mindestens 50% des mit Flüssigkeit gefüllten Reaktorvolumens muß eine rückvermischungsfreie Rohrströmungscharakteristik herrschen. (Einzelheiten zur Strömungscharakteristik in einem Reaktor siehe Walter Brötz, Grundriß der chemischen Reaktionstechnik, Verlag Chemie, Weinheim, 1958, Seite 300 ff.)

Damit ist nicht ausgeschlossen, daß im Reaktor im untergeordneten Maße auch andere Strömungscharakteristiken, wie z. B. turbulente Strömung, vorliegen können. Von der Rohrströmung abweichende Strömungsverhältnisse können insbesondere im Bereich der Einspeisung der Einsatzstoffe auftreten.

Der für das erfindungsgemäße Verfahren gebräuchliche Reaktor ist ein senkrecht stehender, zylindrischer Rohrreaktor. Das Verhältnis von Länge/Durchmesser eines solchen Reaktors kann von 500/1 bis 3/1 betragen, liegt jedoch vorzugsweise in einem Bereich von 50/1 bis 5/1.

Die Einspeisung der Einsatzstoffe (Olefin, Alkanol, Kohlenmonoxid, Katalysator) erfolgt zweckmäßigerweise am Eingang, d. h. am Boden des Reaktors. Zum Einspeisen können bekannte Vorrichtungen, wie z. B. Mehrstoffdüsen, Siebbodenplatten und ringförmige, parallel zum Boden angeordnete Rohrleitungen mit mehreren Austrittsöffnungen, verwendet werden. Um eine bessere Durchmischung der Einsatzstoffe und gegebenenfalls schon vorhandener Reaktionsprodukte zu erreichen, kann im unteren Teil (<als 50% des von Flüssigkeit gefüllten Reaktorvolumens) des Reaktors die Rohrströmung ohne Rückvermischung durch eine andere Strömungscharakteristik ersetzt sein. Dies läßt sich z. B. durch den Einbau von konzentrischen Innenrohren erreichen. Bei Verwendung eines Innenrohres ist es zweckmäßig, daß dieses nicht über das untere Drittel des mit Flüssigkeit gefüllten Reaktorvolumens hinausragt und daß die Einsatzstoffe mit einer Geschwindigkeit eingedüst werden, die die Ausbildung eines Flüssigkeitsumlaufes um das Innenrohr herum gewährleistet.

Die Abführung der im Reaktor freiwerdenden Reaktionswärme kann sowohl durch in den Reaktionsraum eingebaute Kühleinrichtungen als auch durch Mantelkühlung längs des Reaktors erfolgen. Insbesondere dann, wenn im unteren Teil des Reaktors rückvermischte Strömungsverhältnisse vorliegen, kann es zweckmäßig sein, eine Abführung der anfallenden Reaktionswärme durch das Einspeisen kalter Einsatzstoffe ($T_{Einsatzstoffe} < T_{Reaktionsmischung}$) herbeizuführen.

Als besonders günstig hat es sich beim vorliegenden Verfahren erweisen, wenn man das Reaktionsgemisch nach der Reaktion gegebenenfalls mit einem sauerstoffhaltigen Gas behandelt, das nicht umgesetzte Alkanol und Olefin, den Promotor sowie die Reaktionsprodukte destillativ entfernt, den dabei anfallenden Destillationsrückstand in Pyridin und/oder einem nicht-ortho-substituierten Alkylpyridin aufnimmt, die so erhaltene cobalthaltige Suspension mit einem Gemisch aus Kohlenmonoxid und Wasserstoff, das 10 bis 90 Volumenprozent Wasserstoff enthält, bei einer Temperatur von 100 bis 250°C und einem Druck von mindestens 50 bar behandelt und die hierdurch erhaltene Katalysatorlösung wieder in den Prozeß zurückführt.

Die folgenden Beispiele erläutern das erfindungsgemäße Verfahren.

### Vergleichsbeispiel A

Ein Gemisch aus 29,5 Gewichtsteilen Cobaltnaphthenat mit einem Gehalt von 10 Gewichtsprozent Cobalt und 46,5 Gewichtsteilen $\gamma$-Picolin wird nach Präformierung durch Umsetzung mit Synthesegas (aus gleichen Teilen $H_2$ und CO) bei 170°C, 160 bar und einer mittleren Verweilzeit von 0,5 Stunden zusammen mit 168 Gewichtsteilen eines statistischen Isomerengemisches innenständiger n-Dodecene ($\alpha$-Anteil <1%) und 64 Gewichtsteilen Methanol sowie Kohlenmonoxid über eine Mischdüse am Boden eines senkrecht stehenden zylindrischen Reaktors zugeführt.

Durch ein im Inneren des Reaktors konzentrisch angeordnetes Leitrohr, das über dem Reaktorboden beginnt und unterhalb der Flüssigkeits-Standhöhe endet, wird schlaufenförmiger Flüssigkeitsumlauf um das Leitrohr im Inneren des Reaktors erzeugt. Die Eintrittsgeschwindigkeit der Reaktionskomponenten ist so hoch eingestellt, daß neben einer intensiven Dispergierung der gasförmigen Komponente die umlaufende Flüssigkeitsmenge durch den Impulseintrag etwa 10mal so hoch ist wie die zugeführte Menge.

Der Umsatz erfolgt bei einer Temperatur von 180°C und einem Druck von 170 bar bei einer mittleren Verweilzeit von 1,9 Stunden, wobei die entstehende Reaktionswärme durch eingehängte Kühlrohre abgeführt wird.

Bei diesen Bedingungen werden 63,0% des eingesetzten Olefins umgesetzt mit einer Selektivität von 97%, bezogen auf die Esterbildung, und einem n-Ester-Anteil von 74%.

### Beispiel 1

Vergleichsbeispiel A wird wiederholt mit der Ausnahme, daß der Reaktor, in dem die Hydrocarboxylierung durchgeführt wird, ohne Leitrohr, d. h. bei weitgehender Vermeidung der Rückvermischung betrieben wird. Der dabei erzielte Olefinumsatz beträgt 80,4% bei einer Esterselektivität von 98% und einem n-Ester-Anteil von 74%.

### Beispiel 2

Die Reaktionsbedingungen hinsichtlich Temperatur, Druck und der Einsatzmengen werden wie bei Beispiel 1 eingestellt. Der obere Teil des Leitrohres gemäß Vergleichsbeispiel A wird soweit gekürzt, daß die Flüssigkeit nur im unteren Teil des Reaktors, der ca. 30% des Gesamtreaktionsvolumens ausmacht, umläuft und zwar mit einer Umwälzmenge, die 10mal so hoch ist wie die zugeführte Menge.

Dabei wird ein Olefin-Umsatz von 78,9% erzielt mit 98,5% Selektivität zu Estern mit einem n-Anteil von 75%.

Vergleichsbeispiel B

Ein Gemisch aus 10 Gewichtsteilen Cobaltnaphthenat mit einem Gehalt von 10 Gewichtsprozent Cobalt und 44,7 Gewichtsteile Pyridin wird nach Präformierung unter Bedingungen wie in Vergleichsbeispiel A zusammen mit 81 Gewichtsteilen n-Octen(1) und 32,3 Gewichtsteilen Methanol sowie Kohlenmonoxid über die Mischdüse am Boden eines Reaktors zugeführt. Ausführung und Betriebsweise des Reaktors waren die gleichen wie in Vergleichsbeispiel A.

Der Umsatz erfolgte bei einer Temperatur von 150°C und einem Druck von 135 bar bei einer mittleren Verweilzeit von 1,3 Stunden. Bei diesen Bedingungen wird ein Olefin-Umsatz von 64,6% erzielt mit einer Selektivität von 98% und einem n-Ester-Anteil von 84%.

Beispiel 3

Vergleichsbeispiel B wird wiederholt mit der Ausnahme, daß der Reaktor, in dem die Hydrocarboxylierung durchgeführt wird, ohne Leitrohr, d. h. bei weitgehender Vermeidung der Rückvermischung betrieben wird. Dabei werden 77,3% des eingesetzten Olefins umgesetzt mit einer Selektivität zu Estern von 98%, deren n-Anteil 84,5% beträgt.

Beispiel 4

Die Reaktionsbedingungen hinsichtlich Temperatur, Druck und der Einsatzmengen werden wieder wie bei Beispiel 3 eingestellt. Das Leitrohr gemäß Vergleichsbeispiel A wird von oben her soweit gekürzt, daß die Flüssigkeit nur im unteren Teil des Reaktors, der ca. 45% des Gesamtreaktorinhalts ausmacht, umläuft und zwar mit einer Umwälzmenge, die 10mal so hoch ist wie die zugeführte Menge. Dabei ergibt sich ein Olefin-Umsatz von 76,6% mit 98,3% Selektivität bezüglich Ester-Bildung und einem n-Ester-Anteil von 84,7%.

**Patentansprüche**

1. Verfahren zur kontinuierlichen Herstellung von Alkylestern gesättigter aliphatischer Carbonsäuren durch Umsetzung von mindestens 8 Kohlenstoffatome enthaltenden Olefinen mit Kohlenmonoxid und Alkanol in Gegenwart eines Katalysators bestehend aus einer Cobaltverbindung und einem Promotor aus der Gruppe Pyridin und/oder nicht-ortho-substituierter Alkylpyridine bei erhöhtem Druck und erhöhter Temperatur, bei dem die Einsatzstoffe von Anfang an im gewünschten Verhältnis in den Reaktor eingespeist werden, dadurch gekennzeichnet, daß im Reaktor die Strömungscharakteristik so eingestellt wird, daß Rohrströmung ohne Rückvermischung vorherrscht.

2. Verfahren nach Anspruch 1, dadurch gekennzeichnet, daß man die Einsatzstoffe insgesamt am Eingang des Reaktors einspeist.

3. Verfahren nach den Ansprüchen 1 und 2, dadurch gekennzeichnet, daß man im Eingangsbereich des Reaktors (<50% des Reaktionsraumes) gezielt eine rückvermischte Strömung einstellt.

4. Verfahren nach den Ansprüchen 1 bis 3, dadurch gekennzeichnet, daß man die als Katalysatorkomponente benutzte Cobaltverbindung vor dem Einspeisen in den Reaktor unter Verwendung des Promotors als Reaktionsmedium bei erhöhtem Druck und erhöhter Temperatur einer Behandlung mit einem Gemisch aus Kohlenmonoxid und Wasserstoff unterwirkt.

**Claims**

1. A process for the continuous production of an alkyl ester of a saturated aliphatic carboxylic acid by reacting an olefin of at least 8 carbon atoms with carbon monoxide and an alkanol at elevated pressure and elevated temperature in the presence of a catalyst comprising a cobalt compound and a promoter selected from pyridine and non-ortho-substituted alkylpyridines, the starting materials being fed into the reactor in the desired ratio from the beginning, characterised in that the flow characteristics in the reactor are adjusted to be such that tubular flow predominates without back-mixing.

2. A process according to claim 1, characterised in that the starting materials are fed in entirely at the reactor inlet.

3. A process according to claim 1 or 2, characterised in that a back-mixed flow is deliberately created in the inlet zone of the reactor (<50% of the reaction space).

4. A process according to any of claims 1 to 3, characterised in that, prior to being fed into the reactor, the cobalt compound used as catalyst component is subjected to a treatment with a mixture of carbon monoxide and hydrogen at an elevated pressure and elevated temperature, using the promoter as the reaction medium.

**Revendications**

1. Procédé de préparation continue d'esters d'alkyle d'acides carboxyliques aliphatiques saturés, par réaction d'oléfines contenant au moins 8 atomes de carbone sur le monoxyde de carbone et un alcanol en présence d'un catalyseur formé d'un composé de cobalt et d'un activeur du groupe comprenant la pyridine et/ou les alkyl-pyridines non-substituées en ortho, à pression élevée et à température élevée, dans lequel on délivre dès le début les matières premières au réacteur dans le rapport désiré, caractérisé par le fait que dans le réacteur, on règle la caractéristique d'écoulement de façon telle que l'écoulement tubulaire sans mélange rétrograde prédomine.

2. Procédé selon la revendication 1, caractérisé par le fait que l'on introduit les matières premières dans leur ensemble à l'entrée du réacteur.

3. Procédé selon les revendications 1 et 2, caractérisé par le fait que dans la région d'entrée du réacteur (50% du volume de réaction), on établit à dessein un écoulement mélangé de façon rétrograde.

4. Procédé selon les revendications 1 à 3, caractérisé par le fait que l'on soumet le composé de cobalt utilisé comme constituant de catalyseur, avant l'introduction dans le réacteur, avec utilisation de l'activeur comme milieu de réaction, à un traitement à pression élevée et à température élevée par un mélange de monoxyde de carbone et d'hydrogène.